# FASCICULE DE BREVET EUROPEEN

(11) **EP 0 764 435 B1**
(45) Date de publication et mention de la délivrance du brevet: **04.11.1998**
(21) Numéro de dépôt: 96401817.0
(22) Date de dépôt: 23.08.1996
(51) Int. Cl.: A61K 7/00, A61K 7/13, A61K 7/48

(54) **Composition pour la teinture des fibres kératiniques, contenant un antagoniste de substance P**
Zusammensetzungen zum Färben von Keratinfasern, die einen Antagonisten der Substanz P enthalten
Composition for dyeing keratinic fibres containing a substance P antagonist

(30) Priorité: 19.09.1995 FR 9510979
(43) Date de publication de la demande: 26.03.1997
(73) Titulaire: L'OREAL, 75008 Paris (FR)
(72) Inventeur: De Lachararriere, Olivier, 75015 Paris (FR); Breton, Lionel, 78000 Versailles (FR); Loussouarn, Geneviève, 92110 Clichy (FR)
(74) Mandataire: Lhoste, Catherine

(56) Documents cités:
- EP-A- 0 612 525
- EP-A- 0 680 749
- EP-A- 0 716 850
- DATABASE WPI Week 9216 Derwent Publications Ltd., London, GB; AN 92-127243 XP002006031 & JP-A-04 069 324 (YAMAHATSU SANGYO KK) , 4 Mars 1992
- DATABASE WPI Week 9013 Derwent Publications Ltd., London, GB; AN 90096015 XP002006032 & JP-A-02 048 519 (HOYU KK) , 19 Février 1990

## Description

La présente invention se rapporte une composition notamment cosmétique, et plus particulièrement pour la teinture des fibres kératiniques, contenant au moins un antagoniste de substance P pour diminuer voire éliminer les effets irritants des colorants et/ou pigments et/ou précurseurs de colorants contenus dans une telle composition. Elle se rapporte aussi à un procédé de teinture des fibres kératiniques.

Il existe principalement deux types de coloration des fibres kératiniques. La coloration directe mettant en oeuvre des colorants directs et/ou des pigments qui sont des molécules colorées conférant aux fibres une couleur temporaire s'estompant après quelques shampooings, et la coloration dite "coloration d'oxydation" mettant en oeuvre des précurseurs de colorants d'oxydation et un agent oxydant qui confère aux fibres une couleur tenace.

Ces deux types de coloration nécessitent l'application sur les fibres kératiniques de compositions contenant, dans un support approprié pour la teinture, les divers colorants directs et/ou pigments et/ou précurseurs de colorants d'oxydation en présence d'un agent oxydant.

Ces colorants, pigments et précurseurs de colorants peuvent entraîner des irritations notamment chez certaines personnes, et cet effet irritant est d'autant plus ressenti par le sujet traité que celui-ci a le cuir chevelu sensible.

La demanderesse a découvert que l'utilisation d'antagonistes de substance P pouvait permettre d'obtenir un effet préventif et/ou curatif de l'irritation due aux colorants, pigments et/ou précurseurs de colorants.

La substance P est un élément chimique polypeptidique élaboré et libéré par les terminaisons nerveuses. Elle fait partie de la famille des tachykinines. La substance P intervient notamment dans la transmission de la douleur et dans des maladies du système nerveux central tels que l'anxiété, la schizophrénie, dans des maladies respiratoires et inflammatoires, dans des maladies gastro-intestinales, dans des maladies rhumatismales et dans certaines maladies dermatologiques telles que l'eczéma.

Personne jusqu'à ce jour n'avait envisagé d'utiliser des antagonistes de substance P en application préalable avant une teinture ou en association avec des matières colorantes en vue d'éliminer l'effet irritant de ces dernières. Toutefois, le document selon l'article 54(3) EP-A-716 850 au nom du demandeur décrit des dérivés particuliers de l'éthylène diamine comme antagonistes de substance P, associés à des aminophénols ou à la paraphénylène diamine et ses dérivés reconnus comme produits à effet secondaire irritant.

Aussi, la présente invention a pour objet une composition notamment cosmétique contenant dans un milieu cosmétiquement acceptable au moins un antagoniste de substance P et au moins un actif à effet secondaire irritant, caractérisée en ce que l'actif à effet secondaire irritant est un colorant, un pigment et/ou un précurseur de colorant, le colorant étant différent d'un aminophénol, de la paraphénylène diamine et de ses dérivés lorsque l'antagoniste de substance P est un dérivé de l'éthylène diamine ayant deux groupements benzyle substitués ou non en méta et séparés par le groupement éthylène diamine.

La composition selon l'invention est notamment une composition de teinture des fibres kératiniques, et en particulier une composition de teinture des cheveux humains.

Aussi, la présente invention a encore pour objet une composition de teinture des fibres kératiniques contenant, dans un milieu approprié pour la teinture, un colorant, un pigment et/ou un précurseur de colorant à effet secondaire irritant, caractérisée en ce qu'elle contient en outre au moins un antagoniste de substance P.

L'antagoniste de substance P peut se trouver dans la composition de teinture elle-même, ou bien dans une composition appliquée sur les fibres kératiniques avant l'application de la composition de teinture.

Par conséquent, la présente invention a aussi pour objet un produit de teinture des fibres kératiniques, comprenant une première composition contenant au moins un antagoniste de substance P et une seconde composition contenant, dans un milieu approprié pour la teinture, un colorant, un pigment et/ou un précurseur de colorant à effet secondaire irritant, les deux compositions étant destinées à être appliquées l'une après l'autre.

Selon un mode particulier de réalisation de l'invention, la première et la seconde compositions sont conditionnées séparément sous forme d'un kit, selon un agencement bien connu de l'homme du métier, notamment dans le domaine pharmaceutique.

Aussi, la présente invention a encore pour objet un kit pour teinture des fibres kératiniques et en particulier des fibres kératiniques humaines telles que les cheveux, comprenant une première composition contenant au moins un antagoniste de substance P et une seconde composition contenant, dans un milieu approprié pour la teinture, un colorant, un pigment et/ou un précurseur de colorant à effet secondaire irritant, les deux compositions, destinées compositions, destinées à être appliquées l'une après l'autre, étant conditionnées séparément.

La présente invention a aussi pour objet un procédé de teinture des fibres kératiniques et en particulier des fibres kératiniques humaines telles que les cheveux, caractérisé en ce que l'on applique sur les fibres une composition contenant au moins un antagoniste de substance P et au moins un colorant, un pigment et/ou précurseur de colorant à effet secondaire irritant, dans un milieu approprié pour la teinture.

La présente invention a en outre pour objet un procédé de teinture des fibres kératiniques et en particulier des fibres kératiniques humaines telles que les cheveux, caractérisé en ce que l'on applique sur les fibres une première composition contenant au moins un antagoniste de substance P, puis une seconde composition contenant au moins un colorant, un pigment et/ou précurseur de colorant à effet secondaire irritant, dans un milieu approprié pour la teinture.

La seconde composition est appliquée sur les fibres kératiniques un certain temps après l'application de la première composition, cet intervalle de temps étant de manière avantageuse de 5 à 10 minutes.

La présente invention a encore pour objet l'utilisation d'au moins un antagoniste de substance P dans une composition pour la teinture des fibres kératiniques, pour diminuer et/ou éliminer les effets irritants des colorants et/ou pigments et/ou précurseurs de colorants.

Pour qu'une substance soit reconnue comme un antagoniste de substance P, elle doit répondre à la caractéristique suivante :
- avoir une activité pharmacologique antagoniste de la substance P, c'est-à-dire induire une réponse pharmacologique cohérente dans au moins l'un des deux tests suivants :
- la substance antagoniste doit diminuer l'extravasation du plasma au travers de la paroi vasculaire induite par la capsaïcine ou par une stimulation nerveuse antidromique, ou bien
- la substance antagoniste doit provoquer une inhibition de la contraction des muscles lisses induites par l'administration de substance P.

L'antagoniste de substance P peut en outre avoir une affinité sélective pour les récepteurs NK1 des tachykinines.

L'antagoniste de substance P de l'invention peut être fonctionnel ou réceptoriel, c'est-à-dire inhiber la synthèse et/ou la libération de substance P, ou empêcher sa fixation et/ou moduler son action. Il peut être choisi parmi les composés connus comme antagonistes de substance P, notamment les peptides ou les dérivés azotés non peptidiques, et plus précisément ceux comportant un hétérocycle azoté ou un atome d'azote lié directement ou indirectement à un cycle benzènique, les sels de cobalt et les sels des éléments de la colonne II A de la classification périodique. Il peut être également choisi parmi des extraits d'origine végétale et/ou bactérienne.

Ainsi, on peut utiliser dans l'invention par exemple comme peptide antagoniste de substance P le sendide et le spantide II.

Le sendide correspond à la formule :
Tyr D-Phe Phe D-His Leu Met NH₂
dans laquelle :
Tyr représente la tyrosine,
D-Phe représente la D-phénylalanine,
Phe représente la phénylalanine,
D-His représente la D-histidine
Leu représente la leucine,
Met représente la méthionine.

Le spantide II correspond à la formule :
D-NicLys Pro 3-Pal Pro D-Cl₂Phe Asn D-Trp Phe D-Trp Leu Nle NH₂
dans laquelle :
D-NicLys représente le nicotinate de D-lysine,
Pro représente la proline,
3-Pal représente la 3-pyridyl-alanine,
D-Cl₂Phe représente la D-dichlorophénylalanine,
Asn représente l'asparagine,
D-Trp représente le D-tryptophane,
Phe représente la phénylalanine,
Leu représente la leucine,
Nle représente la nor-leucine.

On peut également utiliser dans l'invention comme peptide antagoniste de substance P les peptides décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques utilisables dans l'invention sont notamment des composés comprenant un atome d'azote lié directement ou indirectement à un cycle benzénique ou contenu dans un hétérocycle.

Comme composé hétérocyclique, on peut utiliser dans l'invention ceux décrits dans les documents suivants : EP-A-360390, EP-A-429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/1 8878, WO-A-91/1 8899, WO-A-92/12151 , WO-A-92/15585, WO-A-92/17449, WO-A-92/20676, WO-A-93/00330, WO-A-93/00331, WO-A-93/01159, WO-A-93/01169, W0-A-93/01170, WO-A-93/06099, WO-A-93/09116. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle ou un dérivé d'isoindole.

Comme composés comportant un atome d'azote lié directement ou indirectement à un noyau benzénique, on peut citer ceux décrits dans les documents suivants : EP-A-522808 et WO-A-93/01165.

Les sels des éléments de la colonne II A de la classification périodique utilisables dans l'invention peuvent être des sels de béryllium ou de métal alcalino-terreux, notamment de strontium, de magnésium. De façon avantageuse, ce sel est un sel de strontium.

Les extraits d'origine bactérienne utilisables dans l'invention peuvent être des extraits d'au moins une bactérie filamenteuse non photosynthétique.

Comme extrait d'origine végétale utilisable dans l'invention, on peut citer notamment ceux provenant *d'Iris germanica, d'Iris florentina, d'Iris pallida,* de *Crocus versicolor,* de *Romulea bulbucodium* ou encore de *Gladiolus communis.* Plus particulièrement selon l'invention, on utilise un extrait végétal issu d'une iridacée et préférentiellement du matériel végétal *d'Iris pallida.* Toute méthode d'extraction connue de l'homme du métier peut être utilisée pour préparer l'extrait contenu dans la composition selon l'invention. On peut, en particulier, citer les extraits alcooliques, notamment éthanoliques ou encore hydroalcooliques. On peut également utiliser un extrait préparé par la méthode décrite dans la demande de brevet français n° 95-02379 déposée par la demanderesse.

Dans les compositions selon l'invention, l'antagoniste de substance P est utilisé de préférence en une quantité allant de 0,000001 à 30 % en poids par rapport au poids total de la composition, et en particulier en une quantité allant de 0,0001 à 10 % en poids par rapport au poids total de la composition.

L'antagoniste de substance P joue essentiellement un rôle sur les colorants ayant un effet irritant, notamment les colorants d'oxydation, les colorants non irritants étant par ailleurs présents dans la composition selon l'invention pour permettre d'atteindre les nuances souhaitées.

Les colorants directs utilisables selon l'invention sont choisis parmi les colorants directs classiquement employés pour la coloration directe des fibres kératiniques. Parmi ceux-ci, on peut citer à titre d'exemple, les dérivés nitrés de la série benzénique, les colorants azoïques, les colorants anthraquinoniques, les indamines, indoanilines et indophénols, les colorants acides tels que ceux référencés au Color Index 3ème édition, les colorants naturels tels que la Lawsone. Ces colorants directs peuvent éventuellement porter des groupements sulfoniques ou cationiques de façon à améliorer leur solubilité dans le milieu utilisé pour la teinture.

Les pigments utilisables selon l'invention peuvent être choisis parmi les pigments minéraux ou organiques que l'on utilise de façon classique en cosmétique.

Parmi les pigments minéraux, on peut citer à titre d'exemple le dioxyde de titane (rutile ou anastase) éventuellement traité en surface et codifié dans le Color Index sous la référence CI 77891 les oxydes de fer noir, jaune, rouge et brun, codifiés sous les références CI 77499, 77492, 77491 ; le violet de manganèse (CI 77742) ; le bleu outremer (CI 77007) ; l'oxyde de chrome hydraté (CI 77289) ; le bleu ferrique (CI 77510).

Parmi les pigments organiques, on peut citer à titre d'exemple, le pigment yellow 3 vendu notamment sous la dénomination commerciale Jaune Covanor W 1603 par le société Wackherr (CI 11710), le D & C red n° 19 (CI 45170), le D & C red n° 9 (CI 15585), le D & C red n° 21 (CI 45380), le D & C orange n° 4 (CI 15510), le D & C orange n^{o} 5 (CI 45370), le D & C red n° 27 (CI 45410), le D & C red n° 13 (CI 15630), le D & C red n° 7 (CI 15850-1), le D & C red n° 6 (CI 15850-2), le D & C yellow n° 5 (CI 19140), le D & C red n° 36 (CI 12085), le D & C orange n° 10 (CI 45425), le D & C yellow n° 6 (CI 15985), le D & C red n° 30 (CI 73360), le D & C red n° 3 (CI 45430), le noir de carbone (CI 77266), et les laques à base de carmin de cochenille (CI 75470).

On peut également utiliser des pigments nacrés qui peuvent être notamment choisis parmi les pigments nacrés blancs tels que le mica recouvert d'oxyde de titane, l'oxyde de bismuth ; les pigments nacrés colorés tels que le mica titane avec des oxydes de fer, le mica titane avec du bleu ferrique ou de l'oxyde de chrome, le mica titane avec un pigment organique de type précipité, ainsi que ceux à base d'oxychlorure de bismuth.

Les précurseurs de colorants d'oxydation utilisables selon l'invention dans les compositions destinées à la coloration d'oxydation sont connus en eux-mêmes. On peut se référer plus particulièrement à ZVIAK, Sciences des traitements capillaires 1988, pages 235 à 287. Il s'agit plus particulièrement de diamines ou aminophénols comportant des groupements fonctionnels amino et hydroxy en position para ou ortho. Ces précurseurs de colorants d'oxydation, encore appelés bases d'oxydation, sont des composés incolores ou faiblement colorés qui une fois mélangés à des produits oxydants, au moment de l'emploi, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, choisis notamment parmi les métadiamines aromatiques, les métaaminophénols et les métadiphénols.

Les compositions de teinture peuvent également contenir des précurseurs indoliques générant des pigments de type mélanique sous l'action d'un agent oxydant. Ces précurseurs indoliques peuvent constituer selon les cas une base d'oxydation ou un coupleur. Ces précurseurs indoliques sont plus particulièrement décrits dans les demandes de brevet et brevets français FR-A-2 593 061, 2 593 062, 2 595 245, 2 606 636, 2 636 237 et les demandes de brevet européen EP-A-425 345, EP-A-424 261. Les précurseurs indoliques préférés sont choisis parmi le 5,6-dihydroxyindole et ses dérivés et les 6- et 7-monohydroxyindoles.

Le milieu approprié pour la teinture (ou support) de la composition est généralement un milieu aqueux constitué par de l'eau ou un mélange d'eau et d'au moins un solvant organique utilisé pour solubiliser les composés qui ne seraient pas suffisamment solubles dans l'eau.

Parmi ces solvants, on peut citer à titre d'exemple, les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylène-glycol, le monoéthyléther et le monométhyléther du diéthyléneglycol ; ainsi que les alcools aromatiques comme l'alcool benzylique ou le phénoxyéthanol ; les produits analogues et leurs mélanges.

Lorsqu'ils sont présents, les solvants représentent de préférence 1 à 50 % en poids du poids total de la composition de teinture et encore plus préférentiellement de 5 à 30 % en poids.

L'antagoniste de substance P permet en outre de réduire l'effet irritant de certains de ces solvants.

Les compositions de teinture conformes à l'invention peuvent également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement, des agents filmogènes, des agents conservateurs, des agents opacifiants, des filtres solaires.

Le pH de la composition appliquée sur les cheveux est de préférence compris entre 3 et 11. Il est ajusté à la valeur désirée au moyen d'agents alcalinisants habituellement utilisés en teinture des fibres kératiniques, tels que l'ammoniaque, les carbonates alcalins, les alcanolamines telles que par exemple les mono-, di- et triéthanolamines ainsi que leurs dérivés, les hydroxydes de sodium ou de potassium ou au moyen d'agents acidifiants classiques tels que des acides minéraux ou organiques comme par exemple les acides chlorhydrique, tartrique, citrique, lactique et orthophosphorique.

Les compositions de teinture conformes à l'invention peuvent se présenter sous des formes diverses, telles que sous forme de liquides, de crèmes, de gels, ou sous toute autre forme appropriée pour réaliser une teinture des fibres kératiniques, et notamment des cheveux humains.

Lorsque les compositions de teinture sont utilisées pour la coloration d'oxydation, celles-ci sont mélangées au moment de l'emploi avec une composition contenant, dans un milieu approprié pour la teinture, au moins un agent oxydant.

L'agent oxydant est choisi parmi les agents oxydants classiquement utilisés en coloration d'oxydation et de préférence parmi le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante est tel qu'après mélange avec la composition de teinture, le pH de la composition résultante appliquée sur les fibres kératiniques est de préférence compris entre 3 et 11. Il est ajusté à la valeur désirée à l'aide d'agents acidifiants ou éventuellement alcalinisants bien connus de l'état de la technique, tels que décrits ci-dessus.

Le mélange de la composition de teinture et de la composition oxydante est ensuite appliqué sur les fibres kératiniques dans les mêmes conditions que précédemment.

Les quantités des différents constituants des compositions selon l'invention sont celles classiquement utilisées dans les domaines considérés.

Les exemples qui suivent sont destinés à illustrer l'invention sans pour autant en limiter la portée.

### Exemple 1 ; Composition de teinture par oxydation

| | |
|---|---|
| - Paraphénylènediamine | 0,4 g |
| - 4-hydroxyindole | 0,1 g |
| - Résorcine (coupleur additionnel) | 0,3 g |
| - Chlorure de strontium | 5 g |
| - Alcool oléique polyglycérolé à 2 moles de glycérol | 4 g |
| - Alcool oléique polyglycérolé à 4 moles de glycérol, à 78 % de matières actives (M.A.) | 5,69 g M.A. |
| - Acide oléique | 3 g |
| - Amine oléique à 2 moles d'oxyde d'éthylène vendue sous la dénomination commerciale ETHOMEEN O12 par la société AKZO | 7 g |
| - Laurylamino succinamate de diéthylaminopropyle, sel de sodium, à 55 % de M.A. | 3 g M.A. |
| - Alcool oléique | 5 g |
| - Diéthanolamide d'acide oléique | 12 g |
| - Propylèneglycol | 3,5 g |
| - Alcool éthylique | 7 g |
| - Dipropylèneglycol | 0,5 g |
| - Monométhyléther de propylèneglycol | 9 g |
| - Métabisulfite de sodium en solution aqueuse, à 35 % de M.A. | 0,455 g M.A. |
| - Acétate d'ammonium | 0,8 g |
| - Antioxydant, séquestrant | q.s. |
| - Parfum, conservateur | q.s. |
| - Ammoniaque à 20 % de NH₃ | 10 g |
| - Eau q.s.p. | 100 g |

Au moment de l'emploi, on mélange la composition obtenue avec une quantité égale d'une composition constituée par une solution d'eau oxygénée à 20 volumes (6 % en poids).

### Exemple 2 :

On a réalisé la même composition que dans l'exemple 1, en remplaçant les 5 g de chlorure de stronium par 0,05 g de Spantide II.

### Exemple 3:

On a réalisé la même composition que dans l'exemple 1, en remplaçant les 5 g de chlorure de stronium par 5 g d'un extrait *d'Iris pallida* préparé de la manière suivante :

Des cellules indifférenciées *d'Iris pallida* cultivées *in vitro* en conditions axéniques sont récupérées après culture en Erlenmeyer ou en fermenteur par filtration sur tamis de 50µm. A 55 g de matière fraîche ainsi obtenue, on ajoute 27,5 ml d'eau déminéralisée. L'ensemble est broyé au Turax à 24000 T/min durant 1 minute à 4°C (bain de glace). Le broyat est centrifugé à 4°C. Le surnageant est filtré à 0,22 µm (filtration stérilisante).

L'extrait ainsi préparé est conservé à 4°C. Il renferme environ 15 g de matière sèche par litre.

### Exemple 4 : Kit de teinture

### Première composition : lotion à appliquer avant la teinture

| | |
|---|---|
| - Chlorure de strontium | 7 g |
| - Alcool éthylique | 10 g |
| - Eau | qsp 100 g |

### Deuxième composition : composition de teinture

| | |
|---|---|
| - Paraphénylénediamine | 0,4 g |
| - 4-hydroxyindole | 0,1 g |
| - Résorcine (coupleur additionnel) | 0,3 g |
| - Alcool oléique polyglycérolé à 2 moles de glycérol | 4 g |
| - Alcool oléique polyglycérolé à 4 moles de glycérol, à 78 % de matières actives (M.A.) | 5,69 g M.A. |
| - Acide oléique | 3 g |
| - Amine oléique à 2 moles d'oxyde d'éthylène vendue sous la dénomination commerciale ETHOMEEN O12 par la société AKZO | 7 g |
| - Laurylamino succinamate de diéthylaminopropyle, sel de sodium, à 55 % de M.A. | 3 g M.A. |
| - Alcool oléique | 5 g |
| - Diéthanolamide d'acide oléique | 12 g |
| - Propylèneglycol | 3,5 g |
| - Alcool éthylique | 7 g |
| - Dipropylèneglycol | 0,5 g |
| - Monométhyléther de propylèneglycol | 9 g |
| - Métabisulfite de sodium en solution aqueuse, à 35 % de M.A. | 0,455 g M.A. |
| - Acétate d'ammonium | 0,8 g |
| - Antioxydant, séquestrant | q.s. |
| - Parfum, conservateur | q.s. |
| - Ammoniaque à 20 % de NH₃ | 10 g |
| - Eau q.s.p. | 100 g |

On applique tout d'abord la première composition sur les cheveux, puis après un laps de temps de 5 minutes, on applique la composition de teinture.

## Revendications

1. Composition notamment cosmétique contenant dans un milieu cosmétiquement acceptable au moins un antagoniste de substance P et au moins un actif à effet secondaire irritant, caractérisée en ce que l'actif à effet secondaire irritant est un colorant, un pigment et/ou un précurseur de colorant, le colorant étant différent d'un aminophénol, de la paraphénylène diamine et de ses dérivés lorsque l'antagoniste de substance P est un dérivé de l'éthylène diamine ayant deux groupements benzyle substitués ou non en méta et séparés par le groupement éthylène diamine.

2. Composition de teinture des fibres kératiniques contenant, dans un milieu approprié pour la teinture, au moins un colorant, un pigment et/ou un précurseur de colorant à effet secondaire irritant, caractérisée en ce qu'elle contient en outre au moins un antagoniste de substance P, le colorant étant différent d'un aminophénol, de la paraphénylène diamine et de ses dérivés lorsque l'antagoniste de substance P est un dérivé de l'éthylène diamine ayant deux groupements benzyle substitués ou non en méta et séparés par le groupement éthylène diamine.

3. Composition selon la revendication 1 ou 2, caractérisée en ce que l'antagoniste de substance P est choisi parmi les peptides, les composés comprenant au moins un hétérocycle azoté, les sels de cobalt et les sels des éléments de la colonne II A de la classification périodique, les extraits d'origine végétale, les extraits d'origine bactérienne.

4. Composition selon la revendication 3, caractérisée en ce que le peptide est le sendide ou le spantide II.

5. Composition selon la revendication 3, caractérisée en ce que le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle, un dérivé d'isoindole.

6. Composition selon la revendication 3, caractérisée en ce que le sel de l'élément de la colonne II A de la classification périodique est choisi parmi les chlorures, carbonates, borates, nitrates, acétates, hydroxydes, sulfates, les sels d'acides de fruits et les sels d'acides aminés de baryum, calcium, magnésium, strontium et/ou de béryllium.

7. Composition selon la revendication 6, caractérisée en ce que le sel est un sel de strontium.

8. Composition selon la revendication 3, caractérisée en ce que l'extrait d'origine végétale est un extrait d'iridacée.

9. Composition selon l'une quelconque des revendications précédentes, caractérisée en ce que l'antagoniste de substance P est utilisé en une quantité allant de 0,000001 à 30 % en poids par rapport au poids total de la composition.

10. Composition selon l'une quelconque des revendications précédentes, caractérisée en ce que l'antagoniste de substance P est utilisé en une quantité allant de 0,0001 à 10% en poids par rapport au poids total de la composition.

11. Composition selon l'une quelconque des revendications précédentes, caractérisée par le fait que le colorant est choisi parmi les colorants directs.

12. Composition selon la revendication précédente, caractérisée par le fait que le colorant direct est choisi parmi les dérivés nitrés de la série benzénique, les colorants azoïques, les colorants anthraquinoniques, les indamines, les indoanilines, les indophénols, les colorants acides, les colorants naturels.

13. Composition selon l'une quelconque des revendications précédentes, caractérisée par le fait que le pigment est choisi parmi les pigments minéraux, organiques ou nacrés.

14. Composition selon l'une quelconque des revendications précédentes, caractérisée par le fait que le précurseur de colorant est choisi parmi les diamines et les aminophénols comportant des groupements fonctionnels amino et hydroxy en position para ou ortho.

15. Composition selon l'une quelconque des revendications précédentes, caractérisée par le fait qu'elle est destinée à la coloration d'oxydation et contient en outre au moins un coupleur choisi parmi les métadiamines aromatiques, les métaaminophénols et les métadiphénols.

16. Composition selon l'une quelconque des revendications précédentes, caractérisée par le fait qu'elle est destinée à la coloration d'oxydation et contient au moins un précurseur indolique.

17. Composition selon la revendication précédente, caractérisée par le fait que le précurseur indolique est choisi parmi le 5,6-dihydroxyindole et ses dérivés et les 6- et 7-monohydroxyindoles.

18. Composition selon l'une quelconque des revendications précédentes, caractérisée par le fait que le milieu est un milieu aqueux constitué par de l'eau ou un mélange d'eau et d'un solvant organique.

19. Composition selon l'une quelconque des revendications précédentes, caractérisée par le fait que la composition renferme en outre au moins un adjuvant choisi parmi les agents tensio-actifs, les polymères, les agents épaississants, les agents antioxydants, les agents de pénétration, les agents séquestrants, les parfums, les tampons, les agents dispersants, les agents de conditionnement, les agents filmogènes, les agents conservateurs, les agents opacifiants, les filtres solaires.

20. Produit de teinture des libres kératiniques, comprenant une première composition contenant au moins un antagoniste de substance P et une seconde composition contenant, dans un milieu approprié pour la teinture, un colorant, un pigment et/ou un précurseur de colorant à effet secondaire irritant, les deux compositions étant destinées à être appliquées l'une après l'autre.

21. Produit selon la revendication 20, caractérisé en ce que l'antagoniste de substance P est un sel de strontium.

22. Procédé de teinture des libres kératiniques et en particulier des libres kératiniques humaines telles que les cheveux, caractérisé en ce que l'on applique sur les libres une composition contenant au moins un antagoniste de substance P et au moins un colorant, un pigment et/ou précurseur de colorant à effet secondaire irritant, dans un milieu approprié pour la teinture.

23. Procédé de teinture des fibres kératiniques et en particulier des fibres kératiniques humaines telles que les cheveux, caractérisé en ce que l'on applique sur les fibres une première composition contenant au moins un antagoniste de substance P, puis une seconde composition contenant au moins un colorant, un pigment et/ou précurseur de colorant à effet secondaire irritant, dans un milieu approprié pour la teinture.

24. Procédé selon la revendication 22 ou 23, caractérisé par le fait que la composition de teinture est mélangée à une composition oxydante avant application sur les fibres kératiniques.

25. Procédé selon l'une quelconque des revendications 22 à 24, caractérisé en ce que l'antagoniste de substance P est choisi parmi les peptides et les composés comprenant au moins un hétérocycle azoté, les sels de cobalt et les sels des éléments de la colonne II A de la classification périodique, les extraits d'origine végétale, les extraits d'origine bactérienne.

26. Procédé selon l'une quelconque des revendications 22 à 25, caractérisé en ce que l'antagoniste de substance P est un sel de strontium.

27. Procédé selon l'une quelconque des revendications 22 à 25, caractérisé en ce que l'antagoniste de substance P est un extrait d'iridacée.

28. Kit pour teinture des fibres kératiniques et en particulier des fibres kératiniques humaines telles que les cheveux, comprenant une première composition contenant au moins un antagoniste de substance P et une seconde composition contenant, dans un milieu approprié pour la teinture, un colorant, un pigment et/ou un précurseur de colorant à effet secondaire irritant, les deux compositions, destinées à être appliquées l'une après l'autre, étant conditionnées séparément.

29. Utilisation d'au moins un antagoniste de substance P dans une composition pour la teinture des fibres kératiniques, pour diminuer et/ou éliminer les effets irritants des colorants et/ou pigments et/ou précurseurs de colorants, le colorant étant différent d'un aminophénol, de la paraphénylène diamine et de ses dérivés lorsque l'antagoniste de substance P est un dérivé de l'éthylène diamine ayant deux groupements benzyle substitués ou non en méta et séparés par le groupement éthylène diamine.

## Claims

1. Composition, in particular a cosmetic composition, containing, in a cosmetically acceptable medium, at least one substance P antagonist and at least one active agent with an irritant side effect, characterized in that the active agent with an irritant side effect is a dye, a pigment and/or a dye precursor, the dye being other than an aminophenol, para-phenylenediamine or its derivatives when the substance P antagonist is an ethylenediamine derivative having two meta-substituted or unsubstituted benzyl groups separated by the ethylenediamine group.

2. Composition for dyeing keratin fibres, containing, in a medium which is suitable for dyeing, at least one dye, one pigment and/or one dye precursor with an irritant side effect, characterized in that it also contains at least one substance P antagonist, the dye being other than an aminophenol, para-phenylenediamine or its derivatives when the substance P antagonist is an ethylenediamine derivative having two meta-substituted or unsubstituted benzyl groups separated by the ethylenediamine group..

3. Composition according to Claim 1 or 2, characterized in that the substance P antagonist is chosen from peptides, compounds comprising at least one nitrogenous heterocycle, cobalt salts and salts of elements of column II A of the Periodic Table, extracts of plant origin and extracts of bacterial origin.

4. Composition according to Claim 3, characterized in that the peptide is sendide or spantide II.

5. Composition according to Claim 3, characterized in that the compound comprising at least one nitrogenous heterocycle is a 2-tricyclyl-2-aminoethane derivative, a spirolactam derivative, a quinuclidine derivative, an azacyclic derivative, an aminopyrrolidine derivative, a piperidine derivative, an aminoazaheterocycle or an isoindole derivative.

6. Composition according to Claim 3, characterized in that the salt of the element of column II A of the Periodic Table is chosen from the chlorides, carbonates, borates, nitrates, acetates, hydroxides and sulphates, the salts of fruit acids and the amino acid salts of barium, calcium, magnesium, strontium and/or beryllium.

7. Composition according to Claim 6, characterized in that the salt is a strontium salt.

8. Composition according to Claim 3, characterized in that the extract of plant origin is an Iridaceae extract.

9. Composition according to any one of the preceding claims, characterized in that the substance P antagonist is used in an amount ranging from 0.000001 to 30 % by weight relative to the total weight of the composition.

10. Composition according to any one of the preceding claims, characterized in that the substance P antagonist is used in an amount ranging from 0.0001 to 10 % by weight relative to the total weight of the composition.

11. Composition according to any one of the preceding claims, characterized in that the dye is chosen from direct dyes.

12. Composition according to the preceding claim, characterized in that the direct dye is chosen from nitro derivatives of the benzene series, azo dyes, anthraquinone dyes, indamines, indoanilines, indophenols, acidic dyes and natural dyes.

13. Composition according to any one of the preceding claims, characterized in that the pigment is chosen from inorganic, organic or pearlescent pigments.

14. Composition according to any one of the preceding claims, characterized in that the dye precursor is chosen from diamines and aminophenols containing amino and hydroxyl functional groups in the ortho or para position.

15. Composition according to any one of the preceding claims, characterized in that it is intended for oxidation dyeing and also contains at least one coupler chosen from aromatic meta-diamines, meta-aminophenols and meta-diphenols.

16. Composition according to any one of the preceding claims, characterized in that it is intended for oxidation dyeing and contains at least one indole precursor.

17. Composition according to the preceding claim, characterized in that the indole precursor is chosen from 5,6-dihydroxyindole and derivatives thereof and 6- and 7-monohydroxyindoles.

18. Composition according to any one of the preceding claims, characterized in that the medium is an aqueous medium consisting of water or a mixture of water and an organic solvent.

19. Composition according to any one of the preceding claims, characterized in that the composition also includes at least one adjuvant chosen from surfactants, polymers, thickeners, antioxidants, penetration agents, sequestering agents, fragrances, buffers, dispersing agents, packaging agents, film-forming agents, preserving agents, opacifiers and sunscreens.

20. Product for dyeing keratin fibres, comprising a first composition containing at least one substance P antagonist and a second composition containing, in a medium which is suitable for dyeing, a dye, a pigment and/or a dye precursor with an irritant side effect, the two compositions being intended to be applied one after the other.

21. Product according to Claim 20, characterized in that the substance P antagonist is a strontium salt.

22. Process for dyeing keratin fibres and in particular human keratin fibres such as the hair, characterized in that a composition containing at least one substance P antagonist and at least one dye, one pigment and/or one dye precursor with an irritant side effect, in a medium which is suitable for dyeing, is applied to the fibres.

23. Process for dyeing keratin fibres and in particular human keratin fibres such as the hair, characterized in that a first composition containing at least one substance P antagonist is applied to the fibres, followed by a second composition containing at least one dye, one pigment and/or one dye precursor with an irritant side effect, in a medium which is suitable for dyeing.

24. Process according to Claim 22 or 23, characterized in that the dye composition is mixed with an oxidizing composition before application to the keratin fibres.

25. Process according to any one of Claims 22 to 24, characterized in that the substance P antagonist is chosen from peptides and compounds comprising at least one nitrogenous heterocycle, cobalt salts and salts of the elements of column II A of the Periodic Table, extracts of plant origin and extracts of bacterial origin.

26. Process according to any one of Claims 22 to 25, characterized in that the substance P antagonist is a strontium salt.

27. Process according to any one of Claims 22 to 25, characterized in that the substance P antagonist is an Iridaceae extract.

28. Kit for dyeing keratin fibres and in particular human keratin fibres such as the hair, comprising a first composition containing at least one substance P antagonist and a second composition containing, in a medium which is suitable for dyeing, a dye, a pigment and/or a dye precursor with an irritant side effect, the two compositions, intended to be applied one after the other, being packaged separately.

29. Use of at least one substance P antagonist in a composition for dyeing keratin fibres, in order to reduce and/or eliminate the irritant effects of the dyes and/or pigments and/or dye precursors, the dye being other than an aminophenol, para-phenylenediamine or its derivatives when the substance P antagonist is an ethylenediamine derivative having two meta-substituted or unsubstituted benzyl groups separated by the ethylenediamine group.

## Patentansprüche

1. Zusammensetzungen, insbesondere kosmetische Zusammensetzungen, die in einem kosmetisch akzeptablen Medium mindestens einen Antagonisten der Substanz P und mindestens einen Wirkstoff mit reizender Nebenwirkung enthalten, dadurch gekennzeichnet, daß der Wirkstoff mit reizender Nebenwirkung ein Farbstoff und/oder ein Pigment und/oder ein Farbstoffvorprodukt eines Oxidationsfarbstoffes ist, wobei der Farbstoff von Aminophenolen, p-Phenylendiamin und seinen Derivaten verschieden ist, wenn der Antagonist der Substanz P ein Ethylendiaminderivat mit zwei Benzylgruppen ist, die gegebenenfalls in m-Stellung substituiert und durch die Ethylendiamingruppe voneinander getrennt sind.

2. Zusammensetzungen zum Färben von Keratinfasern, die in einem zum Färben geeigneten Medium mindestens einen Farbstoff und/oder ein Pigment und/oder ein Farbstoffvorprodukt eines Oxidationsfarbstoffes mit reizender Nebenwirkung enthalten, dadurch gekennzeichnet, daß sie ferner mindestens einen Antagonisten der Substanz P enthalten, wobei der Farbstoff von Aminophenolen, p-Phenylendiamin und seinen Derivaten verschieden ist, wenn der Antagonist der Substanz P ein Ethylendiaminderivat mit zwei Benzylgruppen ist, die gegebenenfalls in m-Stellung substituiert und durch die Ethylendiamingruppe voneinander getrennt sind.

3. Zusammensetzungen nach Anspruch 1 oder 2, dadurch gekennzeichnet, daß der Antagonist der Substanz P unter den Peptiden, den Verbindungen, die mindestens einen Stickstoffheterocyclus aufweisen, den Cobaltsalzen, den Salzen der Elemente der Gruppe IIA des Periodensystems und den Extrakten pflanzlichen Ursprungs und den Extrakten bakteriellen Ursprungs ausgewählt ist.

4. Zusammensetzungen nach Anspruch 3, dadurch gekennzeichnet, daß das Peptid das Sendide oder Spantide II ist.

5. Zusammensetzungen nach Anspruch 3, dadurch gekennzeichnet, daß die Verbindung mit mindestens einem Stickstoffheterocyclus ein 2-Tricyclyl-2-amino-ethanderivat, Spirolactamderivat, Chinuclidinderivat, azacyclisches Derivat, Aminopyrrolidinderivat, Piperidinderivat, Aminoazacyclus oder Isoindolderivat ist.

6. Zusammensetzungen nach Anspruch 3, dadurch gekennzeichnet, daß das Salz des Elements der Gruppe IIA des Periodensystems unter den Chloriden, Carbonaten, Boraten, Nitraten, Acetaten, Hydroxiden, Sulfaten, den Salzen von Fruchtsäuren und den Salzen von Aminosäuren von Barium, Calcium, Magnesium, Strontium und/oder Beryllium ausgewählt ist.

7. Zusammensetzungen nach Anspruch 6, dadurch gekennzeichnet, daß das Salz ein Strontiumsalz ist.

8. Zusammensetzungen nach Anspruch 3, dadurch gekennzeichnet, daß der Pflanzenextrakt ein Extrakt von Iridaceae ist.

9. Zusammensetzungen nach einem der vorhergehenden Ansprüche, dadurch gekennzeichnet, daß der Antagonist der Substanz P in einem Mengenanteil im Bereich von 0,000001 bis 30 Gew.-%, bezogen auf das Gesamtgewicht der Zusammensetzung, verwendet wird.

10. Zusammensetzungen nach einem der vorhergehenden Ansprüche, dadurch gekennzeichnet, daß der Antagonist der Substanz P in einem Mengenanteil im Bereich von 0,0001 bis 10 Gew.-%, bezogen auf das Gesamtgewicht der Zusammensetzung, verwendet wird.

11. Zusammensetzungen nach einem der vorhergehenden Ansprüche, dadurch gekennzeichnet, daß der Farbstoff unter den Direktfarbstoffen ausgewählt ist.

12. Zusammensetzungen nach dem vorhergehenden Anspruch, dadurch gekennzeichnet, daß der Direktfarbstoff unter den nitrierten Derivaten aus der Benzolgruppe, Azofarbstoffen, Anthrachinonfarbstoffen, Indaminen, Indoanilinen und Indophenolen, sauren Farbstoffen und natürlichen Farbstoffen ausgewählt ist.

13. Zusammensetzungen nach einem der vorhergehenden Ansprüche, dadurch gekennzeichnet, daß das Pigment unter den anorganischen Pigmenten, organischen Pigmenten oder Perlglanzpigmenten ausgewählt ist.

14. Zusammensetzungen nach einem der vorhergehenden Ansprüche, dadurch gekennzeichnet, daß das Farbstoffvorprodukt eines Oxidationsfarbstoffes unter den Diaminen und Aminophenolen, die in o- oder p- Stellung funktionelle Amino- und Hydroxygruppen aufweisen, ausgewählt ist.

15. Zusammensetzungen nach einem der vorhergehenden Ansprüche, dadurch gekennzeichnet, daß sie zum oxidativen Färben verwendet werden und ferner mindestens einen Kuppler enthalten, der unter den aromatischen m-Diaminen, m-Aminophenolen und m-Dihydroxybenzolen ausgewählt ist.

16. Zusammensetzungen nach einem der vorhergehenden Ansprüche, dadurch gekennzeichnet, daß sie zum oxidativen Färben verwendet werden und ferner mindestens ein Indolvorprodukt enthalten.

17. Zusammensetzungen nach dem vorhergehenden Anspruch, dadurch gekennzeichnet, daß das Indolvorprodukt unter 5,6-Hydroxyindol oder seinen Derivaten und den 6- oder 7-Monohydroxyindolen ausgewählt ist.

18. Zusammensetzungen nach einem der vorhergehenden Ansprüche, dadurch gekennzeichnet, daß das Medium ein wäßriges Medium ist, das aus Wasser oder einem Gemisch aus Wasser und einem organischen Lösungsmittel besteht.

19. Zusammensetzungen nach einem der vorhergehenden Ansprüche, dadurch gekennzeichnet, daß sie ferner mindestens einen Hilfsstoff enthalten, der unter den grenzflächenaktive Stoffen, Polymeren, Verdickungsmitteln, Antioxidantien, Penetrationsmitteln, Maskierungsmitteln, Parfums, Puffern, Dispergiermitteln, Konditioniermittel, Filmbildnern, Konservierungsmitteln, Trübungsmitteln und Sonnenschutzfiltern ausgewählt ist.

20. Produkt zum Färben von Keratinfasern, das eine erste Zusammensetzung mit mindestens einem Antagonisten der Substanz P und eine zweite Zusammensetzung umfaßt, die in einen kosmetisch akzeptablen Medium einen Farbstoff, ein Pigment und/oder ein Farbstoffvorprodukt eines Oxidationsfarbstoffes mit reizender Nebenwirkung enthält, wobei die beiden Zusammensetzungen nacheinander aufgebracht werden sollen.

21. Produkt nach Anspruch 20, dadurch gekennzeichnet, daß der Antagonist der Substanz P ein Strontiumsalz ist.

22. Verfahren zum Färben von Keratinfasern und insbesondere menschlichen Keratinfasern, wie dem Haar, dadurch gekennzeichnet, daß auf die Fasern eine Zusammensetzung aufgetragen wird, die mindestens einen Antagonisten der Substanz P und mindestens einen Farbstoff, ein Pigment und/oder ein Farbstoffvorprodukt eines Oxidationsfarbstoffes mit reizender Nebenwirkung in einem zum Färben geeigneten Medium enthält.

23. Verfahren zum Färben von Keratinfasern und insbesondere menschlichen Keratinfasern, wie dem Haar, dadurch gekennzeichnet, daß auf die Fasern eine erste Zusammensetzung, die mindestens einen Antagonisten der Substanz P enthält, und dann eine zweite Zusammensetzung aufgetragen wird, die in einem zum Färben geeigneten Medium mindestens einen Farbstoff, ein Pigment und/oder ein Farbstoffvorprodukt eines Oxidationsfarbstoffes mit reizender Nebenwirkung enthält.

24. Verfahren nach Anspruch 23 oder 24, dadurch gekennzeichnet, daß die Färbemittelzusammensetzung vor dem Auftragen auf die Keratinfasern mit einer oxidierenden Zusammensetzung vermischt wird.

25. Verfahren nach einem der Ansprüche 22 bis 24, dadurch gekennzeichnet, daß der Antagonist der Substanz P unter den Peptiden, den Verbindungen, die mindestens einen Stickstoffheterocyclus aufweisen, den Cobaltsalzen, den Salzen der Elemente der Gruppe IIA des Periodensystems und den Extrakten pflanzlichen Ursprungs und den Extrakten bakteriellen Ursprungs ausgewählt ist.

26. Verfahren nach einem der Ansprüche 22 bis 25, dadurch gekennzeichnet, daß der Antagonist der Substanz P ein Strontiumsalz ist.

27. Verfahren nach einem der Ansprüche 22 bis 25, dadurch gekennzeichnet, daß der Antagonist der Substanz P ein Extrakt von Iridaceae ist.

28. Kits zum Färben von Keratinfasern und insbesondere menschlichen Keratinfasern, wie dem Haar, die eine erste Zusammensetzung, die mindestens einen Antagonisten der Substanz P enthält, und eine zweite Zusammensetzung enthalten, die in einem zum Färben geeigneten Medium mindestens einen Farbstoff, ein Pigment und/oder ein Farbstoffvorprodukt eines Oxidationsfarbstoffes mit reizender Nebenwirkung enthält, wobei die beiden Zusammensetzungen, die nacheinander aufgebracht werden sollen, getrennt voneinander konfektioniert sind.

29. Verwendung mindestens eines Antagonisten der Substanz P in einer Zusammensetzung zum Färben von Keratinfasern, um den durch die Farbstoffe, Pigmente und Farbstoffvorprodukte von Oxidationsfarbstoffen hervorgerufenen Reizungen vorzubeugen oder abzuhelfen, wobei der Farbstoff von Aminophenolen, p-Phenylendiamin und seinen Derivaten verschieden ist, wenn der Antagonist der Substanz P ein Ethylendiaminderivat mit zwei Benzylgruppen ist, die gegebenenfalls in m-Stellung substituiert und durch die Ethylendiamingruppe voneinander getrennt sind.
